# EUROPEAN PATENT APPLICATION

(11) **EP 4 717 232 A1**
(43) Date of publication of application: **01.04.2026**
(21) Application number: 24810275.8
(22) Date of filing: 15.05.2024
(51) Int. Cl.: A61F 2/24, A61F 2/95, A61F 2/82, A61F 2/844

(54) **STENT ASSEMBLING STRUCTURE, CATHETER ASSEMBLY AND DELIVERY SYSTEM**

(30) Priority: 22.05.2023 CN 202310582006
(71) Applicant: Shanghai MicroPort CardioFlow Medtech Co., Ltd., Shanghai 201203 (CN)
(72) Inventor: YU, Wenxia, Shanghai 201203 (CN); ZHANG, Pinghai, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2024/093328
(87) International publication number: WO 2024/240028

(57) **Abstract**

This application relates to a stent load structure. A securing member comprises a securing bore axially extending therethrough. The securing bore is configured for insertion of an inner core tube therein. The securing member has a distal securing portion configured to secure a valvular stent. A guide member comprises a guide bore axially extending therethrough, which is configured for insertion of the inner core tube therein. A proximal end of the guide member is coupled to a distal end of the securing member so that the guide bore is brought into communication with the securing bore. The guide member that is sleeved over the inner core tube is configured to support the valvular stent. Guided by the guide member, the valvular stent can be more easily loaded. Moreover, the valvular stent is directly supported by the guide member, instead of by the inner core tube that is thin and has a relatively small diameter, so that the guide member may fill the gap between the valvular stent and the inner core tube. Thus, the thin inner core tube can be supported and thereby protected from possible flexing during its delivery, or during loading of the valvular stent, ensuring smooth passage of the guide wire and robust release of the valvular stent.

## Description

### TECHNICAL FIELD

The present application relates to the field of medical devices and, in particular, to a stent load structure, a catheter assembly and a delivery system.

### BACKGROUND

Heart valve replacement involves using a delivery system to deliver, for example, a mitral, tricuspid, pulmonary or similar valve to a target site, such as the aortic root, and then release the valve there after its correct positioning has been confirmed, replacing the native valve. This has become a highly sought-after, cutting-edge technique in the field of valvular heart disease, marking a milestone in minimally invasive interventional treatment of aortic valve disease. This technique can be used to treat aortic valve disease without thoracotomy, heartbeat stoppage or other highly traumatic intervention as may be necessary for traditional surgical procedures.

At present, valvular stents commonly used in heart valve replacement are self-expanding ones, and most systems for delivering self-expanding valvular stents incorporate a multi-layer catheter, for example, consisting of an inner core tube, an intermediate inner catheter and an outer sheath. The inner core tube comprises a guide wire lumen, and a self-expanding valvular stent is preoperatively loaded in a crimped configuration between the outer sheath and the inner core tube. Most such valvular stents, when expanded, comprise a flower shape, that is, the tines at the leading ends of the valvular stent open in a petal manner, which are designed primarily to provide support after expansion, except for two symmetrical ones of them that are intended for engagement with the inner core tube to hook the self-expanding valvular stent.

However, a portion of the inner core tube for loading of a self-expanding valvular stent thereon is usually very thin and small in outer diameter and tends to be undesirably flexed during the preoperative loading and intraoperative retrieval, affecting the passage of a guide wire therethrough and hence hindering robust release of the self-expanding valvular stent. Moreover, the tines of the self-expanding valvular stent are prone to piling and twisting, which can easily cause scratches or other damage to the outer sheath of the delivery system. All of these are detrimental to the success of the procedure.

### SUMMARY OF THE INVENTION

In view of the above, it is necessary to provide a stent load structure, a catheter assembly and a delivery system, which overcome at least one of the above-described problems.

Accordingly, there is provided herein a stent load structure comprising:
a securing member comprising a securing bore axially extending therethrough, wherein the securing bore is configured for insertion of an inner core tube therein, and wherein the securing member comprises a distal securing portion configured to secure a valvular stent;
a guide member comprising a guide bore axially extending therethrough, wherein the guide bore is configured for insertion of the inner core tube therein, wherein a proximal end of the guide member is coupled to a distal end of the securing member so that the guide bore is brought into communication with the securing bore, and wherein the guide member over the inner core tube is configured to support the valvular stent; and
a separation member provided on the guide member and configured to space apart and support a plurality of branched portions of the valvular stent.

In one embodiment, the stent load structure comprises:
a sealing element, wherein the sealing element is disposed on the guide member and is configured to seal a gap between the guide bore of the guide member and an outer wall of the inner core tube; or
a protrusion, wherein the sealing element is disposed on an inner wall of the guide bore and is configured for sealing contact with the outer wall of the inner core tube.

In one embodiment, the guide member comprises a distal guide section and a proximal guide section, each of the distal guide section and the proximal guide section is a cylindrical tube, and wherein a maximum outer diameter of the distal guide section is smaller than a minimum outer diameter of the proximal section.

Additionally or alternatively, the securing member comprises a distal securing section and a proximal securing section, wherein each of the distal securing section and the proximal securing section is a cylindrical tube, and wherein a maximum outer diameter of the distal securing section is smaller than a minimum outer diameter of the proximal section.

In one embodiment, the separation member is provided on the proximal guide section.

Additionally or alternatively, a transition guide section is connected between the distal and proximal guide sections, wherein the transition guide section is a cylindrical tube, and wherein an outer diameter of the transition guide section gradually increases from a distal end to a proximal end.

In one embodiment, the separation member comprises a plurality of unit separators that are arranged circumferentially around an outer wall of the proximal guide section, and wherein a unit separation space is provided between adjacent unit separators and is configured to receive the branched portion of the valvular stent.

In one embodiment, the unit separator is a separation plate, and wherein a height of the unit separator with respect to the outer wall of the proximal guide section increases from a distal end to a proximal end.

In one embodiment, a first connecting member and a second connecting member are provided at the proximal end of the guide member and the distal end of the securing member, respectively, wherein the proximal end of the guide member is coupled to the distal end of the securing member by the first and second connecting member.

Alternatively, the proximal end of the guide member is adhesively secured to the distal end of the securing member.

In one embodiment, the first connecting member is a socket provided at the proximal end of the guide member, and the second connecting member is a plug provided at the distal end of the securing member, which is inserted into the socket.

Alternatively, the first connecting member is a threaded bore provided at the proximal end of the guide member, and the second connecting member is a threaded stem provided at the distal end of the securing member, which threads with the threaded bore.

Also provided herein is a catheter assembly comprising:
an inner core tube comprising a guide wire lumen axially extending therethrough, wherein the guide wire lumen is configured for passage of a guide wire therethrough;
an inner catheter comprising a catheter lumen axially extending therethrough, wherein the inner core tube is disposed in the catheter lumen, and wherein a distal end of the inner core tube extends out of a distal end of the inner catheter;
the stent load structure, wherein the section of the inner core tube that extends out of the inner catheter is inserted in the securing bore of the securing member and the guide bore of the guide member; and
an outer sheath comprising a sheath lumen axially extending therethrough, wherein each of the inner core tube, the inner catheter and the stent load structure is disposed in the outer sheath.

Also provided herein a delivery system comprising:
the stent load structure; or the catheter assembly.

In the stent load structure, the catheter assembly and the delivery system, the guide member can be sleeved over the inner core tube which may be thin and have a smaller diameter. Guided by the guide member, the valvular stent can be more easily loaded over the inner core tube. Moreover, the valvular stent is directly supported by the guide member, instead of by the thin inner core tube, such that the guide member may fill the gap between the valvular stent and the inner core tube. Thus, the thin inner core tube can be supported and thereby protected from possible flexing during its delivery, or during loading of the valvular stent, ensuring smooth passage of the guide wire and robust release of the valvular stent.

### BRIEF DESCRIPTION OF THE DRAWINGS

Fig. 1 schematically illustrates a stent load structure in a loaded configuration according to an embodiment of the present application.
Fig. 2 schematically illustrates a stent load structure in a loaded configuration according to another embodiment of the present application.
Fig. 3 schematically illustrates a stent load structure in a loaded configuration according to yet another embodiment of the present application.
Fig. 4 schematically illustrates a guide member according to an embodiment of the present application.
Fig. 5 schematically illustrates a guide member according to another embodiment of the present application.
Fig. 6 schematically illustrates a valvular stent in a loaded configuration according to an embodiment of the present application.

### List of Reference Numerals

1000 securing member; 2000 guide member; 3000 inner core tube; 4000 distal guide head; 5000 valvular stent; 6000 inner catheter;
1000a distal securing section; 1000b proximal securing section; 1100 distal securing portion; 1200 first connecting member; 1300 second connecting member;
2000a distal guide section; 2000b proximal guide section; 2000c transition guide section; 2100 sealing element; 2200 protrusion; 2300 unit separator; 2300a unit separation space;
5100 branched portion.

### DETAILED DESCRIPTION

Objects, advantages and features of this application will become more apparent upon reading the following more detailed description of specific embodiments thereof in conjunction with the accompanying drawings. In the following description, numerous details are set forth in order to provide a thorough understanding of the application. However, the present application may be practiced in many other forms than those described herein, and those skilled in the art can make similar improvements without deviating from the spirit of the application. Accordingly, the present application is not limited to the specific embodiments disclosed below.

It will be understood that terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. may be used herein to describe directional or positional relationships based on the orientations shown in the figures. They are intended merely to facilitate and simplify the explanation of the application and do not indicate or imply that the stated components or elements have to comprise, or be constructed or operated in, particular orientations. Therefore, they are not to be construed as limiting the present application.

In addition, as used herein, the terms "first", "second" and the like are intended only for illustration and are not to be construed as denoting or implying relative importance, or as implicitly indicating the number of the referenced items. Accordingly, defining an item with "first" or "second" is an explicit or implicit indication of the presence of at least one such items. As used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with intervening media, or an internal communication or interaction between two elements. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

It should be noted that when a component is referred to as being "secured" to or "disposed" on another component, it may be directly on the other component, or intervening components may be present. When a component is referred to as being "connected" to another component, it can be directly connected to the other component, or intervening components may be present. As used herein, the terms "vertical", "horizontal", "upper", "lower", "left", "right" and the like are merely illustrative and do not represent the only implementation possible.

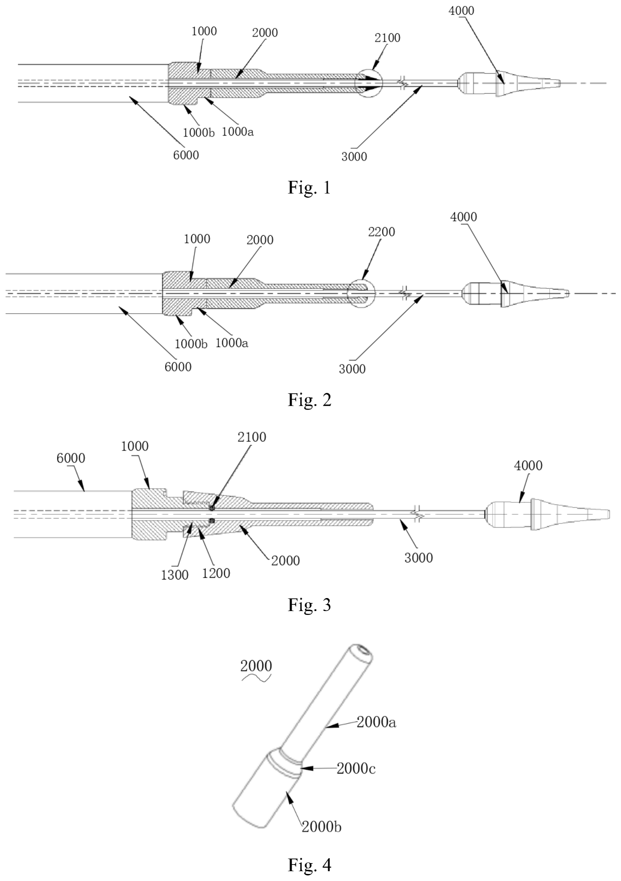

Referring to Fig. 1, an embodiment disclosed herein provides a stent load structure including a securing member 1000 and a guide member 2000. The securing member 1000 comprises a securing bore axially extending therethrough and configured for insertion of the inner core tube 3000 therein. The securing member 1000 has a distal securing portion 1100 configured to secure a valvular stent 5000. The guide member 2000 comprises a guide bore axially extending therethrough and configured for insertion of the inner core tube 3000 therein. A proximal end of the guide member 2000 is coupled to the distal end of the securing member 1000 so that the guide bore is brought into communication with the securing bore. The guide member 2000 over the inner core tube 3000 is configured to support the valvular stent 5000.

The securing member 1000 may be of any of various suitable structures. For example, as shown in Fig. 1, the securing member 1000 may be of a stepped structure. For example, the securing member 1000 may include a distal securing section 1000a and a proximal securing section 1000b, which are both cylindrical tubes. A maximum outer diameter of the distal securing section 1000a is smaller than a minimum outer diameter of the proximal section. Alternatively, the distal securing section 1000a and the proximal securing section 1000b may be otherwise structured, as required for the securing of the valvular stent 5000. Each of them may be of any suitable regular or irregular outer shape, such as prismatic or hemispherical, as long as the distal securing section 1000a and the proximal securing section 1000b of the securing member 1000 constitute a stepped structure. One skilled in the art may select any suitable structure, without departing from the scope of this application.

The distal securing portion 1100 of the securing member 1000 may have any suitable structures capable of securing the valvular stent 5000, such as notches or slots, which are complementary in shape to lugs or other mating structures of the valvular stent 5000. Mating of such structures enables robust loading of the valvular stent 5000. Once the valvular stent 5000 with a valve is secured, the distal securing portion 1100 can restrict, for example, its circumferential and axial movement. One skilled in the art may also realize the loading of the valvular stent 5000 using a similar or known approach, if required, without departing from the scope of the application.

With continued reference to Fig. 1, the guide member 2000 may be sleeved over the inner core tube 3000. The inner core tube 3000, over which the valvular stent 5000 is loaded, may be thin and have a relative small diameter. The guide member 2000 can guide and facilitate the loading of the valvular stent 5000. Moreover, with this arrangement, the valvular stent 5000 is directly supported by the guide member 2000, instead of by the thin inner core tube 3000, and the guide member 2000 fills the gap between the valvular stent 5000 and the inner core tube 3000. Thus, the thin inner core tube 3000 can be supported and thereby protected from possible flexing during its delivery, or during the loading of the valvular stent 5000.

The inner core tube 3000 comprises a guide wire lumen configured for passage of a guide wire therethrough, which is used to guide a delivery system to be advanced thereon during delivery. Specifically, the guide wire may be brought into contact with a wall of a blood vessel, and the delivery system is advanced along the path and direction guided by the by the guide wire. Moreover, during catheter steering, the guide wire will bend passively in response to bending of the inner core tube 3000. Preventing undesirable flexing of the inner core tube 3000 ensures that its guide wire lumen remains open, advantageously ensuring smooth advancement and passage of the guide wire therethrough. Otherwise, flexing of the inner core tube 3000 may lead to the guide wire lumen being locally blocked and closed, disallowing passage of the guide wire therethrough anymore. Preventing flexing of the inner core tube 3000 also facilitates robust release of the valvular stent.

Referring to Figs. 1 to 3, in one embodiment, the stent load structure may include a sealing element 2100, protrusion 2200 or a similar component. Referring to Fig. 1, the sealing element 2100 may be provided on the guide member 2000, for example, at its distal end. The sealing element 2100 may be made of any suitable material with sealing properties and is used to seal possible gap left between the guide bore of the guide member 2000 and an outer wall of the inner core tube 3000, for example, at the distal end of the guide member 2000, as shown in Fig. 1. For example, the sealing element 2100 may be made of an adhesive material such as glue. In this case, in addition to distally sealing any gap between the guide bore and the outer wall of the inner core tube 3000, the adhesive material can also attach the guide member 2000 to the inner core tube 3000, preventing any undesirable relative rotation between them.

With continued reference to Fig. 2, the stent load structure may alternatively include the protrusion 2200. The protrusion 2200 may project from an inner wall of the guide bore and is configured for sealing contact with the outer wall of the inner core tube 3000. Accordingly, the protrusion 2200 is indeed a circumferentially extending annular collar capable of completely sealing any circumferential gap between the inner wall of the guide bore and the outer wall of the inner core tube 3000. In this case, the protrusion 2200 may be simply in sealing contact with the outer wall of the inner core tube 3000, without completely preventing relative movement, for example, particularly axial relative movement of the guide member 2000 and the inner core tube 3000.

With continued reference to Fig. 3, the sealing element 2100 may be alternatively disposed at the proximal end of the guide member 2000, for example, between the securing member 1000 and the guide member 2000. In this case, the sealing element 2100 may be provided as a sealing ring or the like, made of rubber or another material with sealing properties and is used to seal gap between the guide bore of the guide member 2000 and the outer wall of the inner core tube 3000. One skilled in the art may seal such gap between the guide bore of the guide member 2000 and the outer wall of the inner core tube 3000 using a different approach while selectively restricting their relative rotation or not, without departing from the scope of this application.

The proximal end of the guide member 2000 may be coupled to the distal end of the securing member 1000 by various approaches. For example, as shown in Figs. 1 and 2, the proximal end of the guide member 2000 may be adhesively bonded to the distal end of the securing member 1000. In an alternative embodiment, as shown in Fig. 3, a first connecting member 1200 and a second connecting member 1300 are provided at the proximal end of the guide member 2000 and the distal end of the securing member 1000, respectively. In this case, the proximal end of the guide member 2000 may be coupled to the distal end of the securing member 1000 by the first connecting member 1200 and the second connecting member 1300. Specifically, the first connecting member 1200 may be a socket provided at the proximal end of the guide member 2000, and the second connecting member 1300 may be a plug provided at the distal end of the securing member 1000. The plug may be mated and coupled to the socket by being inserted therein. Still alternatively, the first connecting member 1200 may be a threaded bore provided at the proximal end of the guide member 2000, and the second connecting member 1300 may be a threaded stem provided at the distal end of the securing member 1000. The threaded stem may thread with the threaded bore.

The guide member 2000 may be formed by injection molding of a polymer material, such as pebax, silicone, etc. The guide member 2000 may be of any of various suitable structures. For example, as shown in Fig. 4, the guide member 2000 may be of a stepped structure. For example, the guide member 2000 may include a distal guide section 2000a and a proximal guide section 2000b, which are both cylindrical tubes. A maximum outer diameter of the distal guide section 2000ais smaller than a minimum outer diameter of the proximal section. Alternatively, the distal guide section 2000a and the proximal guide section 2000b may be otherwise structured, as required for the supporting of the valvular stent 5000. Each of them may be of any suitable regular or irregular outer shape, such as prismatic or hemispherical, as long as the distal guide section 2000a and the proximal guide section 2000b of the guide member 2000 constitute a stepped structure. One skilled in the art may select any suitable structure, without departing from the scope of this application. Further, with continued reference to Fig. 4, a transition guide section 2000c may be connected between the distal guide section 2000a and the proximal guide section 2000b. The transition guide section 2000c may be a cylinder tube, and an outer diameter of the transition guide section gradually increases from a distal end to a proximal end.

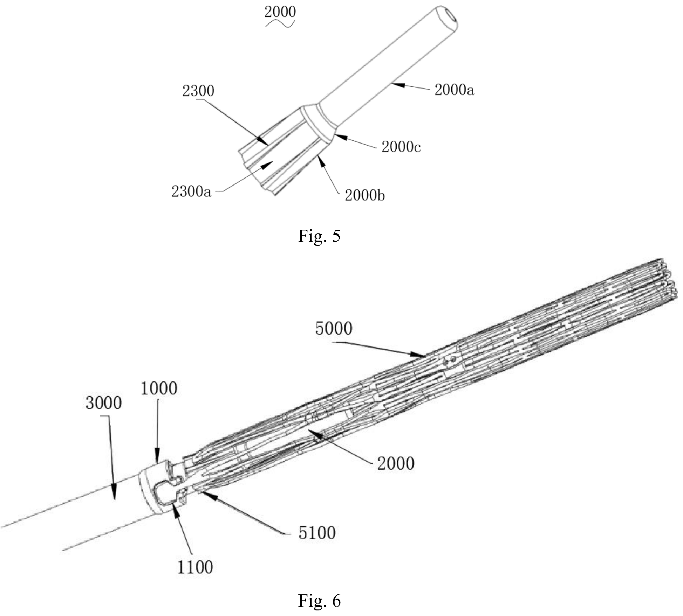

With continued reference to Figs. 5 and 6, in one embodiment, in order to better support the valvular stent 5000 without piling or twisting of branched portions 5100 thereof, the proximal guide section 2000b may be provided with a separation member, and the valvular stent 5000 may be switchable between an expanded configuration and a crimped configuration. Fig. 6 shows the valvular stent 5000 in the crimped configuration. The branched portions 5100 of the valvular stent 5000 may be tines branched off from one end of the valvular stent 5000. The valvular stent 5000 may be loaded in the crimped configuration on the securing member 1000 and on the guide member 2000 of the stent load structure, with some of the branched portions 5100 being secured by the securing member 1000 and with the remaining ones of the branched portions 5100 being spaced apart by the separation member. Thus, the branched portions 5100 of the valvular stent 5000 can be spaced apart by the separation member while being supported thereon, preventing piling and twisting of the branched portion 5100.

The separation member may be of any of various suitable structures. For example, it may be an integral one-piece member provided on the proximal guide section 2000b, or may include multiple unit structures all provided on the proximal guide section 2000b. Referring to Fig. 5, the separation member may include multiple unit separators 2300 arranged circumferentially around the proximal guide section 2000b on an outer wall thereof so that unit separation spaces 2300a are provided between adjacent unit separators 2300. The branched portion 5100 of the valvular stent 5000 may be received in the unit separation space 2300a so as to be each spaced apart from any other branched portion 5100. This provides guidance to the valvular stent 5000 while preventing twisting, crossing and piling of its branched portions 5100, enabling more robust release and retrieval of the valvular stent 5000.

The unit separator 2300 may be of any suitable structure, and the unit separation space 2300a may be of any suitable shape, as long as the branched portion 5100 can be retained in a stable way. Referring to Fig. 5, in one embodiment, the unit separators 2300 may be shaped like plates comprising an increasing height relative to the outer wall of the proximal guide section 2000b from distal end to proximal end. The unit separators 2300 may also be otherwise structured appropriately, for example, as meshes, protrusions or the like. One skilled in the art may make a suitable choice in accordance with the actual circumstances, without departing from the scope of this application.

Also provided herein is a catheter assembly including an inner core tube 3000, an inner catheter 6000, the stent load structure and an outer sheath. The inner core tube 3000 comprises a guide wire lumen axially extending therethrough, which is configured for passage of a guide wire therethrough. The inner catheter 6000 comprises a catheter lumen axially extending therethrough, and the inner core tube 3000 is disposed in the catheter lumen. The outer sheath comprises a sheath lumen axially extending therethrough, and the inner core tube 3000, the inner catheter 6000 and the stent load structure are all accommodated in the sheath lumen. A distal end of the inner core tube 3000 extends out of a distal end of the inner catheter 6000, and the portion of the inner core tube 3000 extending out of a distal end of the inner catheter 6000 is referred to as a loading section on which a valvular stent 5000 can be loaded, that is, the guide member 2000 indirectly loads the valvular stent 5000 so that the valvular stent 5000 is located between the inner core tube 3000 and the outer sheath. With continued reference to Fig. 1, the inner core tube 3000 may be inserted within both the guide bore of the guide member 2000 and the securing bore of the securing member 1000. The inner core tube 3000 may be distally provided with a distal guide head 4000, which may be of a tapered or similar shape capable of facilitating advancement of the entire catheter assembly.

Also provided herein is a delivery system incorporating the stent load structure or the catheter assembly. The structural details, functioning principles and technical benefits of the stent load structure and the catheter assembly have been described above and are not repeated again for the sake of brevity. For more information in this regard, reference is made to the above description.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present application. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims.

## Claims

1. A stent load structure, comprising:
a securing member comprising a securing bore axially extending therethrough, wherein the securing bore is configured for insertion of an inner core tube therein, and wherein the securing member comprises a distal securing portion configured to secure a valvular stent;
a guide member comprising a guide bore axially extending therethrough, wherein the guide bore is configured for insertion of the inner core tube therein, wherein a proximal end of the guide member is coupled to a distal end of the securing member so that the guide bore is brought into communication with the securing bore, and wherein the guide member over the inner core tube is configured to support the valvular stent; and
a separation member provided on the guide member and configured to space apart and support a plurality of branched portions of the valvular stent.

2. The stent load structure according to claim 1, comprising:
a sealing element, wherein the sealing element is disposed on the guide member and is configured to seal a gap between the guide bore of the guide member and an outer wall of the inner core tube; or
a protrusion, wherein the protrusion is disposed on an inner wall of the guide bore and is configured for sealing contact with the outer wall of the inner core tube.

3. The stent load structure according to claim 1, wherein the guide member comprises a distal guide section and a proximal guide section, wherein each of the distal guide section and the proximal guide section is a cylindrical tube, and wherein a maximum outer diameter of the distal guide section is smaller than a minimum outer diameter of the proximal guide section, and/or
wherein the securing member comprises a distal securing section and a proximal securing section, wherein each of the distal securing section and the proximal securing section is a cylindrical tube, and wherein a maximum outer diameter of the distal securing section is smaller than a minimum outer diameter of the proximal securing section.

4. The stent load structure according to claim 3, wherein the separation member is provided on the proximal guide section, and/or
wherein a transition guide section is connected between the distal and proximal guide sections, wherein the transition guide section is a cylindrical tube, and wherein an outer diameter of the transition guide section gradually increases from a distal end to a proximal end.

5. The stent load structure according to claim 4, wherein the separation member comprises a plurality of unit separators that are arranged circumferentially around an outer wall of the proximal guide section, and wherein a unit separation space is provided between adjacent unit separators and is configured to receive the branched portion of the valvular stent.

6. The stent load structure according to claim 5, wherein the unit separator is a separation plate, and wherein a height of the unit separator with respect to the outer wall of the proximal guide section increases from a distal end to a proximal end.

7. The stent load structure according to claim 1, wherein a first connecting member and a second connecting member are provided at the proximal end of the guide member and the distal end of the securing member, respectively, and wherein the proximal end of the guide member is coupled to the distal end of the securing member by the first and second connecting member, or
wherein the proximal end of the guide member is adhesively secured to the distal end of the securing member.

8. The stent load structure according to claim 7, wherein the first connecting member is a socket provided at the proximal end of the guide member, wherein the second connecting member is a plug provided at the distal end of the securing member, and wherein the plug is inserted into the socket, or
wherein the first connecting member is a threaded bore provided at the proximal end of the guide member, wherein the second connecting member is a threaded stem provided at the distal end of the securing member, and wherein the threaded stem threads with the threaded bore.

9. A catheter assembly, comprising:
an inner core tube comprising a guide wire lumen axially extending therethrough, wherein the guide wire lumen is configured for passage of a guide wire therethrough;
an inner catheter comprising a catheter lumen axially extending therethrough, wherein the inner core tube is disposed in the catheter lumen, and wherein a distal end of the inner core tube extends out of a distal end of the inner catheter;
the stent load structure of any one of claims 1 to 8, wherein a section of the inner core tube that extends out of the inner catheter is inserted in the securing bore of the securing member and the guide bore of the guide member; and
an outer sheath comprising a sheath lumen axially extending therethrough, wherein each of the inner core tube, the inner catheter and the stent load structure is disposed in the outer sheath.

10. A delivery system, comprising:
the stent load structure of any one of claims 1 to 8; or
the catheter assembly of claim 9.
